# EUROPÄISCHE PATENTSCHRIFT

(11) **EP 2 730 427 B1**
(45) Veröffentlichungstag und Bekanntmachung des Hinweises auf die Patenterteilung: **22.07.2015**
(21) Anmeldenummer: 13188000.7
(22) Anmeldetag: 10.10.2013
(51) Int. Cl.: B42C 5/00, B42C 19/08

(54) **Vorrichtung zur Aufnahme einer Überlast in einer Rückenbearbeitungsstation einer Buchbindemaschine**
Device for receiving an overload in a spine processing station of a book binding machine
Dispositif d'absorption d'une surcharge dans une station de traitement de dos d'une machine de reliure de livres

(30) Priorität: 08.11.2012 DE 102012022063
(43) Veröffentlichungstag der Anmeldung: 14.05.2014
(73) Patentinhaber: Müller Martini Holding AG, 6052 Hergiswil (CH)
(72) Erfinder: Montauti, Vittorio, 04107 Leipzig (DE)

(56) Entgegenhaltungen:
- EP-A1- 2 053 004
- EP-A1- 2 133 212
- DE-U1-202004 007 808

## Beschreibung

Die Erfindung betrifft eine Vorrichtung zur Aufnahme einer Überlast in einer Rückenbearbeitungsstation einer Buchbindemaschine gemäß dem Oberbegriff von Anspruch 1.

Aus der EP 2 133 212A1 die eine Vorrichtung nach dem Oberbegriff des Anspruchs 1 offenbart, ist eine Rückenbearbeitungsstation in einer Buchbindemaschine bekannt, bei der die Rücken der in einer Klammer hängend geförderten Buchblöcke zwischen einer ortsfesten Stützrolle und einer verstellbar zu dieser in einer Linearführung angeordneten Pressrolle klemmend hindurch geführt werden, während der jeweilige Buchrücken durch eine Fräseinrichtung bearbeitet wird. Um hierbei im Falle von Buchblöcken, die eine zu große Dicke aufweisen, eine Beschädigung des Spindelantriebs, welcher die Pressrolle in Richtung zum Buchrücken hin verfährt, zu vermeiden, ist dieser an einer Führung aufgenommen, die sich über eine im Abstand seitlich neben der Spindel-Längsachse angeordnete Spiraldruckfeder an einem gestellfesten Maschinenteil abstützt. Durch den Versatz zwischen der Spindel-Längsachse und der Längsachse der Feder ergibt sich zum einen ein vergleichsweise großer Platzbedarf; und zum anderen ergibt sich beim Auftreten einer Überlast durch das Rückstellmoment der Feder ein zusätzliches Moment, welches schräg zur SpindelLängsachse auf diese wirkt und die Spindel relativ zur Bewegungsrichtung verkippt.

Aufgrund der mit dem Verkippen der Führung einhergehenden Positionsabweichungen ist die Anordnung eines hochpräzisen Dickensensors, mit welchem sich die Anzahl der überschüssigen Seiten eines Buchblocks anhand der Auslenkung der Führung relativ zu einem gestellfesten Maschinenteil erfassen lässt, nur mit erheblichen Genauigkeitseinschränkungen möglich.

Demgemäß ist es eine Aufgabe der vorliegenden Erfindung, eine Vorrichtung zur Aufnahme einer Überlast in einer Rückenbearbeitungsstation einer Buchbindemaschine zu schaffen, welche eine kompakte Bauform aufweist und eine hochpräzise Erfassung von Dickenunterschieden der verarbeiteten Buchblöcke ermöglicht.

Diese Aufgabe wird erfindungsgemäß durch die Merkmale von Anspruch 1 gelöst.

Gemäß der Erfindung umfasst eine Vorrichtung zur Aufnahme einer Überlast in einer Rückenbearbeitungsstation einer Buchbindemaschine, in der die Rücken der Buchblöcke zur Bearbeitung durch eine Fräse oder sonstige Rückenbearbeitungsvorrichtung in einer umlaufenden Klammer geförderten, eine ortsfeste Stützrolle und eine dieser gegenüberliegende Pressrolle, zwischen denen die jeweiligen Buchrücken klemmend hindurch geführt werden.

Um den Abstand zwischen der Stützrolle und der Pressrolle auf die jeweilige Seitenzahl des gerade bearbeiteten Buchblocks einzustellen, ist die Pressrolle über eine Linearführung und einen Spindelantrieb linear verfahrbar. Der Spindelantrieb umfasst hierbei eine Gewindespindel, die durch einen Stellantrieb, welcher bevorzugt einen Elektromotor und ein nachgeordnetes Getriebe aufweist, in Abhängigkeit von der jeweiligen Buchdicke in die eine oder die andere Richtung rotiert wird. Auf dem Außengewinde der Gewindespindel sitzt ein Innengewindeelement, das drehfest bevorzugt an einer in seitlichen Führungen geführte Brücke befestigt ist, an der die Pressrolle insbesondere frei drehbar gelagert ist. Um bei einem Überschreiten der durch den jeweiligen Abstand zwischen der Pressrolle und der Stützrolle vorgegebenen Solldicke eines zwischen den Rollen hindurch geführten Buchrückens eine Auslenkung der Pressrolle zu ermöglichen, die durch einen Sensor zur Bestimmung der Seitenzahl des Buches erfassbar ist, sind die Antriebswelle des Stellantriebs und das zugehörige Ende der Gewindespindel über ein Längenausgleichselement drehfest und entlang der Drehachse der Gewindespindel verschiebbar miteinander gekoppelt. Das federelastische Element befindet sich dabei bevorzugt in einem Hohlraum im Inneren der Gewindespindel und erstreckt sich koaxial zu dessen Längsachse. Dabei stützt sich das federelastische Element, welches bevorzugt eine vorgespannte Spiraldruckfeder ist, jedoch auch ein gummielastisches Element oder eine Gasdruckfeder oder dergleichen sein kann, mit seinem ersten Ende an der Antriebswelle und mit seinem zweiten Ende an der Innenseite des Gehäuses der Gewindespindel ab.

Die Erfindung besitzt den Vorteil, dass beim Auftreten einer Überlast und einer damit verbundenen axialen Auslenkung der Gewindespindel nahezu keine Drehmomente auf diese wirken, wodurch eine laterale Bewegung derselben senkrecht zur Längsachse selbst bei einem vergleichsweise großen Gewindespiel ausgeschlossen wird. Hierdurch wird die Möglichkeit eröffnet, am freien Ende der Gewindespindel eine Detektorplatte anzuordnen, welche durch einen hoch präzisen Abstandssensor erfasst wird, um im Falle einer Überlast den Betrag der Auslenkung der Detektorplatte zu erfassen bzw. gleichzeitig oder alternativ die Anzahl der überschüssigen Buchseiten in einem Buchblock zu bestimmen.

Bei der bevorzugten Ausführungsform der Erfindung ist das federelastische Element eine Spiraldruckfeder, die sich zur Verstellung der Vorspannung mit ihrem zweiten Ende an einem in den inneren Hohlraum der Gewindespindel axial einschraubbaren Schraubelement abstützt. Hierdurch ergibt sich der Vorteil, dass sich die zum Auslenken der Gewindespindel erforderliche, auf die Pressrolle wirkende Druckkraft einstellen lässt, wodurch es ermöglicht wird, die Andruckkraft der Pressrolle in Abhängigkeit von den Materialeigenschaften des Buchrückens zu verändern.

Nach einem weiteren der Erfindung zu Grunde liegenden Gedanken kann es jedoch ebenfalls vorgesehen sein, dass das federelastische Element eine Gasdruckfeder oder einen Pneumatikzylinder oder ein gummielastisches Element ist, oder zumindest ein solches enthält. Der Vorteil einer Gasdruckfeder besteht hierbei darin, dass diese eine von der Auslenkung im Wesentlichen unabhängige Andruckkraft erzeugt, und bevorzugt gleichzeitig eine gewisse Dämpfung der Bewegung der Pressrolle bewirkt, was dem Auftreten von Schwingungen entgegenwirkt.

Bei der bevorzugten Ausführungsform der Erfindung wird das dem Längenausgleichselement gegenüberliegende stirnseitige Ende der Gewindespindel durch einen Stopfen verschlossen, welcher mit der Gewindespindel rotiert und welcher eine senkrecht zur Längsachse der Gewindespindel verlaufende Detektionsfläche aufweist. Die Position der Detektionsfläche, die beispielsweise aus einer ebenen Metallplatte besteht, wird durch einen gestellfesten Sensor, insbesondere einen induktiven Sensor, erfasst der aus der Positionsänderung der Detektionsfläche den Betrag der Auslenkung bestimmt, welcher ein Maß für die Anzahl der Buchseiten ist, die bezüglich der Sollseitenzahl des gerade zu bearbeitenden Buchrückens zu viel oder auch zu wenig vorhanden sind. Durch diese Ausgestaltung der erfindungsgemäßen Vorrichtung ergibt sich in Verbindung mit der nahezu nicht vorhandenen lateralen Auslenkung der Gewindespindel beim Auftreten einer Überlast der Vorteil, dass die Anzahl der fehlenden oder überschüssigen Buchseiten mit einer vergleichsweise kostengünstigen Sensoranordnung mit einer sehr hohen Präzision erfasst werden können.

Nach einem weiteren der Erfindung zu Grunde liegenden Gedanken weist das Längenausgleichselement wenigstens eine im Inneren angeordnete Keilnut auf, in welche ein mit der Antriebswelle drehfest verbundenes Keilelement eingreift. Bevorzugt sind zwei oder mehrere Keilnuten und in diese eingreifende Keilelemente vorgesehen, welche sich innerhalb des nach außen hin bevorzugt geschlossenen Gehäuses des Längenausgleichselements um das freie Ende der Antriebswelle herum erstrecken. Hierdurch werden eine zuverlässige Drehmomentübertragung und eine störungsfreie Funktion des Längenausgleichselements gewährleistet.

Bei der bevorzugten Ausführungsform der Erfindung ist am stirnseitigen Ende der Antriebswelle ein Anschlag vorgesehen, der beispielsweise aus einer mit Hilfe einer axialen Schraube am stirnseitigen Ende der Antriebswelle befestigten geschlossenen kreisrunden Scheibe bestehen kann. Der Anschlag wirkt mit einem am Längenausgleichselement vorgesehenen weiteren Anschlag zusammen, um ein axiales Herausbewegen des Keilelements aus der Keilnut zu unterbinden. Der weitere Anschlag kann hierbei beispielsweise durch die stirnseitigen Enden der zwischen den Keilnuten verlaufenden Wandabschnitte des Gehäuses des Längenausgleichselements gebildet werden. Die zuvor beschriebene Ausführungsform bietet den Vorteil, dass sich die Anschläge insbesondere als Einsatz kostengünstig in das Längenausgleichselement integrieren lassen.

Nach einer weiteren Ausführungsform der Erfindung, die eine besonders kompakte Bauweise ermöglicht, stützt sich die Antriebswelle des Stellantriebs über ein ausreichend dimensioniertes Lager, welches auch Axialkräfte aufnehmen kann beispielsweise ein Axialdrucklager an einem gestellfesten Flansch ab, an welchen bevorzugt auch eine Halteplatte angeflanscht ist, die den Stellantrieb, insbesondere das Getriebe des Stelleantriebs, trägt.

Bei der bevorzugten Ausführungsform der Erfindung ist die Gewindespindel in vorteilhafter Weise mit einem Bewegungsgewinde versehen, beispielsweise einem Trapezgewinde d.h. mit einem im Querschnitt trapezförmigen Gewindegang, und das drehfest aufgenommene und relativ zur Gewindespindel verschiebbare Innengewindelement umfasst bei dieser Ausführungsform eine geschlitzte Gewindemutter beispielsweise eine Trapezmutter, deren Gewindespiel über eine Einstellschraube einstellbar ist. Hierdurch lässt sich das Gewindespiel bei auftretendem Verschleiß in kurzer Zeit und mit einfachen Mitteln durch Verstellen der Einstellschraube nachjustieren.

Schließlich ist es bevorzugt vorgesehen, dass die Pressrolle an einer in beidseitigen Führungen verschiebbar geführten Brücke aufgenommen ist, welche mit dem Innengewindeelement mechanisch verbunden ist. Hierdurch ergibt sich eine mechanisch besonders robuste und präzise Zustellung der Pressrolle an die zu bearbeitenden Buchrücken.

Weitere Merkmale der Erfindung sind in den Unteransprüchen beschrieben.

Die Erfindung wird nachfolgend mit Bezug auf die Zeichnung anhand einer bevorzugten Ausführungsform beschrieben.

In der Zeichnung zeigt:

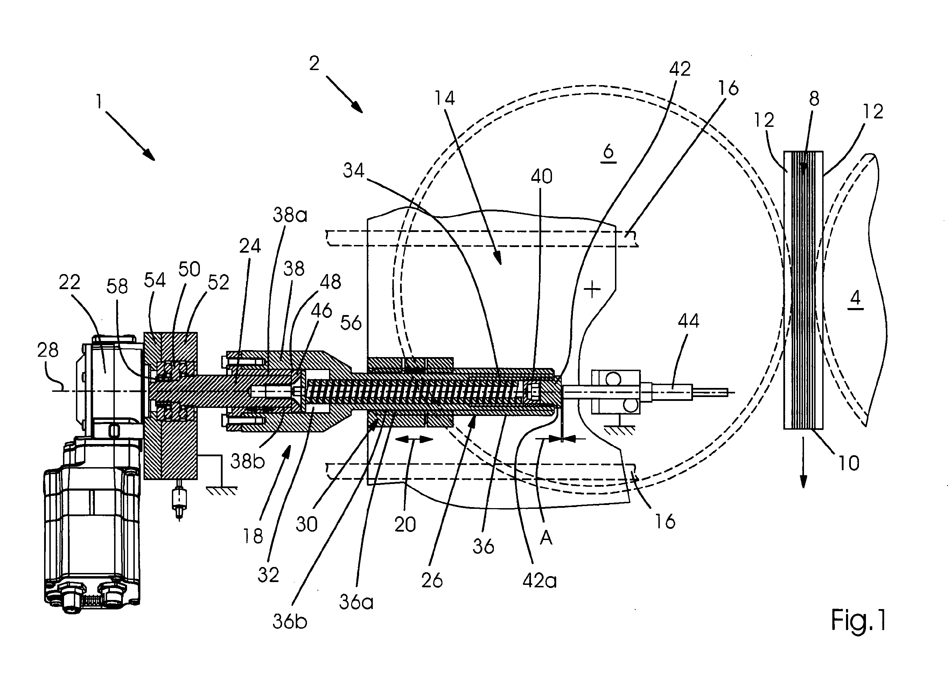

- Fig. 1: eine schematische Seitenansicht der erfindungsgemäßen Vorrichtung mit angedeuteter Stütz- und Pressrolle, zwischen denen ein dargestellter Buchrücken während der Bearbeitung durch eine nicht näher gezeigte Fräseinrichtung hindurchgeführt wird.

Wie in Fig. 1 gezeigt ist, umfasst eine erfindungsgemäße Vorrichtung 1 zur Aufnahme einer Überlast in einer Rückenbearbeitungsstation 2 einer nicht näher gezeigten Buchbindemaschine eine Stützrolle 4 und eine Pressrolle 6, die auf den Rücken 8 eines Buchblocks 10 wirken, wenn dieser bevorzugt hängend in einer Klammer 12 gefördert und durch eine nicht näher gezeigte Fräse bearbeitet wird. Die Pressrolle 6 ist an einer Brücke 14 drehbar aufgenommen, die über eine lediglich schematisch angedeutete Linearführung 16 durch einen Spindelantrieb 18 in Richtung des Doppelpfeils 20 relativ zur gestellfesten Stützrolle 4 verfahrbar ist. Das Verfahren der Pressrolle 6 erfolgt durch einen Stellantrieb 22, der eine Antriebswelle 24 aufweist, welche mit der Gewindespindel 26 des Spindelantriebs 18 über ein Längenausgleichselement 38 drehfest und gleichzeitig entlang der Drehachse 28 der Gewindespindel 26 verschiebbar gekoppelt ist. Auf der Gewindespindel 26 ist ein Innengewindelement 30 angeordnet welches mit der Brücke 14 verbunden ist und diese zusammen mit der Pressrolle 6 in Abhängigkeit von der Drehrichtung der Spindel 26 verfährt, um den Abstand zwischen der Pressrolle 6 und der Stützrolle 4 auf die jeweilige Dicke des gerade verarbeiteten Buchrückens 8 einstellen zu können. Die Gewindespindel 26 ist hierzu auf ihrer Außenseite mit einem Außengewinde 36a versehen, welches in das Innengewinde 36b des Innengewindeelements 30 eingreift.

Um im Falle von Büchern, deren Seitenzahl eine dem Abstand zwischen der Stützrolle 4 und Pressrolle 6 entsprechende Seitenzahl übersteigt, eine Beschädigung der Vorrichtung 1 zu vermeiden und/oder gleichzeitig der Anzahl der überzähligen Buchseiten zu ermitteln, ist innerhalb eines inneren Hohlraums 32 im Inneren der Gewindespindel 26 ein sich koaxial zur Drehachse 28 erstreckendes federelastisches Element in Form einer Spiraldruckfeder 34 angeordnet, welches sich mit seinem ersten Ende über einen Federschutzring 56 an der Antriebswelle 24 und mit seinem zweiten Ende an der Innenseite des Gehäuses der Gewindespindel 26 abstützt. Die Vorspannung der Spiraldruckfeder 34 ist über ein Schraubelement 40 einstellbar, das in den Hohlraum 32 der Gewindespindel axial einschraubbar ist. Am stirnseitigen Ende der Spindel 26 ist ein Stopfen 42 angeordnet, der eine senkrecht zur Drehachse 28 der Gewindespindel verlaufende Detektionsfläche 42a aufweist, die durch einen im geringen Abstand zur Detektionsfläche angeordneten gestellfesten Sensor 44 erfasst wird, um den Abstand A zwischen dem Sensor 44 und der Fläche 42a zu bestimmen.

Wie der Darstellung der Fig. 1 weiterhin entnommen werden kann, weist das Längenausgleichselement 38 wenigstens eine in dessen Innerem geformte Keilnut 38a auf, in welche ein mit der Antriebswelle 24 drehfest verbundenes Keilelement 38b eingreift.

Am stinrnseitigen Ende der Antriebswelle ist ein Anschlag 46 vorgesehen, der mit einem am Längenausgleichselement 38 vorgesehenen weiteren Anschlag 48 zusammenwirkt, um ein axiales Herausbewegen des Keilelements 38b aus der Keilnut 38a durch die vom vorgespannten federelastischen Element 34 erzeugte Druckkraft zu verhindern, welche die Gewindespindel 26 von der Antriebswelle 24 weg drängt.

Wie in Fig. 1 weiterhin gezeigt ist, stützt sich die Antriebswelle 24 des Stellantriebs 22 über ein ausreichend dimensioniertes Lager 50, welches auch Axialkräfte aufnehmen kann, beispielsweise ein Axialdrucklager, oder ein Schrägkugellager oder ein Rillenkugellager an einem gestellfesten Flansch 52 ab, an welchem bevorzugt auch eine Halteplatte 54 für den Stellantrieb 22 angeflanscht ist. Zusätzlich kann zur Sicherung des Lagers 50 ein ringförmiges Blockierelement 56 auf der Antriebswelle 24 befestigt sein. In Figur 1 ist beispielhaft das Lager 50 als Axialdrucklager ausgeführt.

### Bezugszeichenliste

- 1: erfindungsgemäße Vorrichtung
- 2: Rückenbearbeitungsstation
- 4: Stützrolle
- 6: Pressrolle
- 8: Buchrücken
- 10: Buchblock
- 12: Klammer
- 14: Brücke
- 16: Linearführung
- 18: Spindelantrieb
- 20: Doppelpfeil
- 22: Stellantrieb
- 24: Antriebswelle
- 26: Gewindespindel
- 28: Drehachse
- 30: Innengewindeelement
- 32: Hohlraum
- 34: federelastisches Element/Spiraldruckfeder
- 36a: Außengewinde der Gewindespindel
- 36b: Innengewinde des Innengewindeelements
- 38: Längenausgleichselement
- 38a: Keilnut
- 38b: Keilelement
- 40: Schraubelement
- 42: Stopfen
- 42a: Detektionsfläche
- 44: Sensor
- 46: Anschlag
- 48: weiterer Anschlag
- 50: Lager
- 52: gestellfester Flansch
- 54: Halteplatte
- 56: Federschutzring
- 58: ringförmiges Blockierelement
- A: Abstand

## Patentansprüche

1. Vorrichtung (1) zur Aufnahme einer Überlast in einer Rückenbearbeitungsstation (2) einer Buchbindemaschine, in welcher Vorrichtung die Rücken (8) der in einer Klammer (12) geförderten Buchblöcke (10) zur Bearbeitung zwischen einer ortsfesten Stützrolle (4) und einer Pressrolle (6) klemmend hindurch geführt werden, wobei die Pressrolle (6) über eine Linearführung (16) und einen Spindelantrieb (18), welcher eine durch einen Stellantrieb (22) motorisch rotierbare Gewindespindel (26) und ein drehfest aufgenommenes und relativ zur Spindel (26) verschiebbares Innengewindelement (30) umfasst, zur Einstellung der Dicke eines zu bearbeitenden Buchrückens (8) linear verfahrbar ist, und wobei ein mit dem Spindelantrieb (18) gekoppeltes federelastisches Element (34) vorgesehen ist, welches beim Überschreiten einer durch den jeweiligen Abstand zwischen der Pressrolle (6) und der Stützrolle vorgegebenen Solldicke eines zwischen den Rollen (4, 6) geführten Buchrückens (8) eine Auslenkung A der Gewindespindel (26) ermöglicht, welche durch einen Sensor (44) erfassbar ist,
**dadurch gekennzeichnet,dass** der Sensor zur Bestimmung der Seitenzahl des Buchblocks (10) ausgebildet ist und die Antriebswelle (24) des Stellantriebs (22) und das stellantriebseitige Ende der Gewindespindel (26) über ein Längenausgleichselement (38) drehfest und entlang der Drehachse (28) der Gewindespindel (26) verschiebbar miteinander gekoppelt sind, und dass das federelastische Element (34) sich innerhalb eines inneren Hohlraums (32) im Gehäuse der Gewindespindel (26) koaxial zu dessen Drehachse erstreckt und sich mit seinem ersten Ende an der Antriebswelle (24) und mit seinem zweiten Ende an der Innenseite des Gehäuses der Gewindespindel (26) abstützt.

2. Vorrichtung nach Anspruch 1,
**dadurch gekennzeichnet,**
**dass** das federelastische Element (34) eine Spiraldruckfeder ist, die sich zur Verstellung der Vorspannung der Spiraldruckfeder mit ihrem zweiten Ende an einem in den Hohlraum (32) der Gewindespindel (26) axial einschraubbaren Schraubelement (40) abstützt.

3. Vorrichtung nach einem der vorhergehenden Ansprüche,
**dadurch gekennzeichnet,**
**dass** das federelastische Element (34) eine Gasdruckfeder oder einen Pneumatikzylinder oder ein gummielastisches Element umfasst.

4. Vorrichtung nach Anspruch 1 oder 2,
**dadurch gekennzeichnet,**
**dass** das dem Längenausgleichselement (38) gegenüberliegende stirnseitige Ende der Gewindespindel (26) durch einen Stopfen (42) verschließbar ist, welcher eine senkrecht zur Drehachse (28) der Gewindespindel (26) verlaufende Detektionsfläche (42a) aufweist, die durch einen im geringen Abstand zur Detektionsfläche (42a) angeordneten gestellfesten Sensor (44) erfasst wird.

5. Vorrichtung nach Anspruch 4,
**dadurch gekennzeichnet,**
**dass** der Sensor (44) ein induktiver Sensor ist, der den axialen Verfahrweg A der Detektionsfläche (42a) beim Verschieben der Gewindespindel (26) entgegen der Druckkraft des federelastischen Elements (34) im Falle einer Überlast erfasst.

6. Vorrichtung nach einem der vorhergehenden Ansprüche,
**dadurch gekennzeichnet,**
**dass** das Längenausgleichselement (38) wenigstens eine im Inneren angeordnete Keilnut (38a) aufweist, in welche ein mit der Antriebswelle (24) drehfest verbundenes Keilelement (38b) eingreift.

7. Vorrichtung nach einem der vorhergehenden Ansprüche,
**dadurch gekennzeichnet,**
**dass** am stinrnseitigen Ende der Antriebswelle (24) ein Anschlag (46) vorgesehen ist, der mit einem weiteren, am Längenausgleichselement vorgeschlagenen weiteren Anschlag (48) zusammen wirkt, um ein axiales Herausbewegen des Keilelements (38b) aus der Keilnut (38a) durch die vom bevorzugt vorgespannten federelastischen Element (34) erzeugte Druckkraft zu verhindern.

8. Vorrichtung nach einem der vorhergehenden Ansprüche,
**dadurch gekennzeichnet,**
**dass** sich die Antriebswelle (24) des Stellantriebs (22) über ein ausreichend dimensioniertes Lager (50), welches auch Axialkräfte aufnehmen kann an einem gestellfesten Flansch (52) abstützt, an welchem bevorzugt eine Halteplatte (54) für den Stellantrieb (22) angeflanscht ist.

9. Vorrichtung nach einem der vorhergehenden Ansprüche,
**dadurch gekennzeichnet,**
**dass** die Gewindespindel (26) ein Bewegungsgewinde besitzt, und dass das drehfest aufgenommene und relativ zur Spindel (26) verschiebbare Innengewindelement (30) eine geschlitzte Gewindemutter umfasst, deren Gewindespiel bevorzugt über eine Einstellschraube einstellbar ist.

10. Vorrichtung nach einem der vorhergehenden Ansprüche,
**dadurch gekennzeichnet,**
**dass** die Pressrolle (6) an einer in beidseitigen Führungen (16) verschiebbar aufgenommenen Brücke (14) aufgenommen ist, welche mit dem Innengewindeelement (30) mechanisch verbunden ist.

## Claims

1. An apparatus (1) for detecting an overload in a spine-processing station (2) of a bookbinding machine, in which apparatus the spines (8) of the book blocks (10) for processing are guided, conveyed in a clamp (12), between a stationary supporting roller (4) and a pressing roller (6), in which case the pressing roller (6) is movable in linear direction via a linear guide (16) and a spindle drive (18), said spindle drive (18) comprising a rotatable threaded spindle (26), driven by an actuating drive (22), and an internally threaded element (30) mounted for co-rotation with said spindle (26) and displaceable relative to said spindle (26), for the purpose of setting the thickness of a book spine (8) to be processed, and a resilient element (34) coupled to the spindle drive (18) is provided which, when a desired thickness of a book spine guided between the rollers (4, 6) is exceeded as determined by the actual distance between the pressing roller (6) and the supporting roller (4), enables a deflection A of the threaded spindle (26) which is detected by a sensor (44),
**characterized in that** the sensor is designed to determine the number of pages in the book block (10), and the drive shaft (24) of the actuating drive (22) and the end of the threaded spindle (26) on the actuating-drive side are coupled together via a length compensating element (38) for co-rotation and so as to be displaceable along the rotational axis (28) of the threaded spindle (26), **and in that** the resilient element (34) extends within an inner cavity (32) in the housing of the threaded spindle (26) so as to be coaxial with its rotational axis and is supported at its first end on the drive shaft (24) and at its second end against the inside of the housing of the threaded spindle (26).

2. An apparatus according to Claim 1,
**characterized in that**
the resilient element (34) is a spiral pressure spring, which, for the purpose of adjusting the initial tension of said spiral pressure spring, is supported at its second end on a screw element (40) which can be screwed in axial direction into the cavity (32) of the threaded spindle (26).

3. An apparatus according to one of the preceding claims,
**characterized in that**
the resilient element (34) comprises a gas pressure spring or a pneumatic cylinder or a rubber elastic element.

4. An apparatus according to Claim 1 or 2,
**characterized in that**
the end of the threaded spindle (26) on the opposite side to the length compensating element (38) can be closed by a stopper (42), which has a detection surface (42a) that extends perpendicular to the rotational axis (28) of the threaded spindle (26) and is monitored by a fixed sensor (44) mounted a small distance away from said detection surface (42a).

5. An apparatus according to Claim 4,
**characterized in that**
the sensor (44) is an inductive sensor, which monitors the axial travel A of the detection surface (42a) when the threaded spindle (26) is displaced against the pressure of the resilient element (34) in the event of an overload.

6. An apparatus according to one of the preceding claims,
**characterized in that**
the length compensating element (38) is provided with at least one internal keyway (38a), in which a key element (38b) engages, said key element (38b) being connected to the drive shaft (24) for co-rotation therewith.

7. An apparatus according to one of the preceding claims,
**characterized in that**
provided at the front end of the drive shaft (24), there is a limit stop (46), which acts together with another limit stop (48) provided on the length compensating element to prevent an axial movement of the key element (38b) out of the keyway (38a) as a result of the pressure generated by the preferably initially tensioned resilient element (34).

8. An apparatus according to one of the preceding claims,
**characterized in that**
the drive shaft (24) of the actuating drive (22) is supported, via a bearing (50) of adequate size and capable of absorbing axial forces, on a fixed flange (52), onto which preferably a carrier plate (54) for the actuating drive (22) is flange-mounted.

9. An apparatus according to one of the preceding claims,
**characterized in that**
the threaded spindle (26) has a motion thread, **and in that** the internally threaded element (30), mounted for co-rotation with said spindle and displaceable relative to said spindle (26), comprises a slotted threaded nut, its thread clearance preferably being adjustable via an adjusting screw.

10. An apparatus according to one of the preceding claims,
**characterized in that**
the pressing roller (6) is accommodated on a bridge (14), which is mounted so as to be displaceable in bilateral guides (16) and is mechanically connected to the internally threaded element (30).

## Revendications

1. Dispositif (1) pour absorber une surcharge dans un poste (2) de traitement de dos de livres d'une machine de reliure de livres, dispositif dans lequel les dos (8) des corps de livres (10) transportés dans une pince (12) sont, pour le traitement, dirigés avec serrage entre un rouleau d'appui fixe (4) et un rouleau de pressage (6), sachant que le rouleau de pressage (6) peut, au moyen d'un guide linéaire (16) et d'un entraînement à broche (18) qui comprend une broche filetée (26) - rotative de façon motorisée au moyen d'un servomoteur (22) - et un élément à filetage intérieur (30) reçu en solidarité de rotation et mobile en translation par rapport à la broche (26), être déplacé linéairement pour régler l'épaisseur d'un dos de livre (8) à traiter, et sachant qu'il est prévu un élément (34) à élasticité de ressort, qui est couplé à l'entraînement à broche (18) et qui, en cas de dépassement d'une épaisseur de consigne - prédéfinie par la distance respective entre le rouleau de pressage (6) et le rouleau d'appui - d'un dos de livre (8) dirigé entre les rouleaux, permet une excursion A de la broche filetée (26) qui peut être détectée par un capteur (44),
**caractérisé en ce que** le capteur est conçu pour déterminer le nombre de pages du corps de livre (10), et l'arbre d'entraînement (24) du servomoteur (22) et l'extrémité côté servomoteur de la broche filetée (26) sont, au moyen d'un élément (38) de compensation de longueur, couplés entre eux en solidarité de rotation et à translation le long de l'axe de rotation (28) de la broche filetée (26), et **en ce que** l'élément (34) à élasticité de ressort s'étend à l'intérieur d'une cavité intérieure (32) dans le carter de la broche filetée (26) coaxialement à l'axe de rotation de celle-ci, et s'appuie par sa première extrémité contre l'arbre d'entraînement (24) et par sa deuxième extrémité contre le côté intérieur du carter de la broche filetée (26).

2. Dispositif selon la revendication 1, **caractérisé en ce que** l'élément (34) à élasticité de ressort est un ressort de compression spiral qui, afin de régler la précontrainte du ressort de compression spiral, s'appuie par sa deuxième extrémité contre un élément fileté (40) pouvant être vissé axialement dans la cavité (32) de la broche filetée (26).

3. Dispositif selon l'une des revendications précédentes, **caractérisé en ce que** l'élément (34) à élasticité de ressort comprend un ressort de compression à gaz ou un vérin pneumatique ou un élément ayant l'élasticité du caoutchouc.

4. Dispositif selon la revendication 1 ou 2, **caractérisé en ce que** l'extrémité frontale de la broche filetée (26) qui est opposée à l'élément (38) de compensation de longueur peut être fermée par un bouchon (42) qui présente une surface de détection (42a) s'étendant perpendiculairement à l'axe de rotation (28) de la broche filetée (26), surface qui est détectée par un capteur (44) solidaire du bâti, disposé à une faible distance de la surface de détection (42a).

5. Dispositif selon la revendication 4, **caractérisé en ce que** le capteur (44) est un capteur à induction qui détecte la course de déplacement axiale A de la surface de détection (42a) lors de la translation de la broche filetée (26) à l'encontre de la force de compression de l'élément (34) à élasticité de ressort en cas de surcharge.

6. Dispositif selon l'une des revendications précédentes, **caractérisé en ce que** l'élément (38) de compensation de longueur présente au moins une rainure de clavette (38a) disposée à l'intérieur, dans laquelle s'engage un élément formant clavette (38b) relié en solidarité de rotation à l'arbre d'entraînement (24).

7. Dispositif selon l'une des revendications précédentes, **caractérisé en ce qu'**une butée (46) est prévue à l'extrémité frontale de l'arbre d'entraînement (24), butée qui coopère avec une autre butée (48) prévue sur l'élément de compensation de longueur afin d'empêcher un déplacement axial de l'élément formant clavette (38b) hors de la rainure de clavette (38a) sous l'action de la force de compression produite par l'élément (34) à élasticité de ressort, de préférence précontraint.

8. Dispositif selon l'une des revendications précédentes, **caractérisé en ce que** l'arbre d'entraînement (24) du servomoteur (22) s'appuie, par l'intermédiaire d'un palier (50) suffisamment dimensionné qui peut également absorber des forces axiales, contre une bride (52) solidaire du bâti, sur laquelle est de préférence bridée une plaque de maintien (54) pour le servomoteur (22).

9. Dispositif selon l'une des revendications précédentes, **caractérisé en ce que** la broche filetée (26) possède un filetage de déplacement, et **en ce que** l'élément (30) à filetage intérieur, reçu en solidarité de rotation et mobile en translation par rapport à la broche (26), comprend un écrou fileté fendu dont le jeu de filetage peut être de préférence réglé au moyen d'une vis de réglage.

10. Dispositif selon l'une des revendications précédentes, **caractérisé en ce que** le rouleau de pressage (6) est reçu sur un portique (14), qui est reçu à translation dans des guides (16) de part et d'autre et qui est relié mécaniquement à l'élément (30) à filetage intérieur.
